(19) Europäisches Patentamt
European Patent Office
Office européen des brevets

(11) Veröffentlichungsnummer: **0 269 568 B1**

(12) **EUROPÄISCHE PATENTSCHRIFT**

(45) Veröffentlichungstag der Patentschrift: **15.05.91**

(51) Int. Cl.$^5$: **C08G 73/12**, C08F 222/40, C08F 226/06, C08J 5/24

(21) Anmeldenummer: **87810663.2**

(22) Anmeldetag: **16.11.87**

# (54) Polymerisierbare Zusammensetzungen.

(30) Priorität: **22.11.86 GB 8628003**

(43) Veröffentlichungstag der Anmeldung:
**01.06.88 Patentblatt 88/22**

(45) Bekanntmachung des Hinweises auf die Patenterteilung:
**15.05.91 Patentblatt 91/20**

(84) Benannte Vertragsstaaten:
**CH DE FR GB IT LI NL SE**

(56) Entgegenhaltungen:
**EP-A- 0 175 648**
**DE-A- 2 626 832**
**GB-A- 1 512 971**

(73) Patentinhaber: **CIBA-GEIGY AG**
**Klybeckstrasse 141**
**CH-4002 Basel(CH)**

(72) Erfinder: **Banks, Christopher Paul**
**105 Rose Close**
**Saffron Walden Essex CB11 4DU(GB)**
Erfinder: **Irving, Edward, Dr.**
**41, Swaffham Road**
**Burwell Cambridge CB5 0AN(GB)**
Erfinder: **Renner, Alfred, Dr.**
**Marcoup 2**
**CH-3286 Muntelier(CH)**
Erfinder: **Smith, Terence James**
**6A Dickasons Melbourn**
**Royston Hertfordshire SG8 6EL(GB)**

**EP 0 269 568 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft polymerisierbare Zusammensetzungen enthaltend eine Mischung aus einem substituierten Bicyclo[2.2.1]hept-5-en-2,3-dicarbonsäureimid und einem polymerisierbaren Acrylat, deren Polymerisation sowie die Verwendung besagter Gemische.

Allyl- oder methallylsubstituierte Bicyclo[2.2.1]hept-5-en-2,3-dicarbonsäureimide und deren Verwendung zur Herstellung von Polymeren durch Erhitzen auf 180-300°C sind in den EP-A-105,024 und 152,372 beschrieben.

Die Bildung vernetzter Polymerer durch Erhitzen von Imiden dieses Typs in Gegenwart eines kationischen Polymerisationskatalysators wird in der EP-A-155,435 beschrieben.

Wärmehärtbare Mischungen enthaltend ein Epoxidharz, ein Imid vom Typ der in der EP-A-105,024 beschriebenen Verbindungen und einen Härter für das Harz sind aus der EP-A-146,498 bekannt.

Aus dieser letzteren Publikation ist ebenfalls bekannt, dass die härtbaren Gemische Flussmittel, eingeschlossen flüssige Acrylharze, enthalten können. Die üblicherweise als Flussmittel in Epoxidharzzusammensetzungen verwendeten flüssigen Acrylharze sind polymerisierte Acrylharze, die keine polymerisierbaren Acrylgruppen mehr enthalten.

Vernetzbare Polymere unter Verwendung von Imidylbenzoldicarbonsäure-Derivaten sind in DE-A-2 626 832 beschrieben.

Substituierte Bicyclo[2.2.1]hept-5-en-2,3-dicarbonsaureimide und Polymaleinimide enthaltend heisshärtbare Stoffgemische und deren Verwendung sind in EP-A-175 648 beschrieben.

Es wurde nun gefunden, dass Zusammensetzungen enthaltend ein allyl-oder methallylsubstituiertes Bicyclo[2.2.1]hept-5-en-2,3-dicarbonsäureimid und ein polymerisierbares Acrylatmaterial photopolymerisiert werden können und dass polymere Materialien entstehen, die sich zur Herstellung faserverstärker Verbundwerkstoffe oder zur Herstellung von Abbildungen eignen; ferner wurde gefunden, dass sich diese Gemische durch Erhitzen härten lassen und zu Produkten mit ausgezeichneten physikalischen Eigenschaften führen.

Die vorliegende Erfindung betrifft eine polymerisierbare Zusammensetzung enthaltend eine Mischung aus

A) einer Verbindung mit wenigstens einem allyl- oder methalylsubstituierten Bicyclo[2.2.1]hept-5-en-2,3-dicarbonsäureimidrest, und

B) einer Verbindung, die in einer Menge von 5 bis 95 Gew.-%, bezogen auf die Gesamtmenge der Komponenten A und B, vorliegt, mit wenigstens einem polymerisierbaren Rest der Formel I

$$CH_2=C(R^1)-COO- \qquad (I),$$

worin $R^1$ Wasserstoff oder Methyl ist.

Der Imidrest in Komponente A) ist in der Regel am Bicycloheptenring unsubstituiert, von der Allyl- oder Methallylsubstitution abgesehen, oder er ist zusätzlich mit einer Methylgruppe substituiert.

Bevorzugte Imidreste entsprechen der Formel II

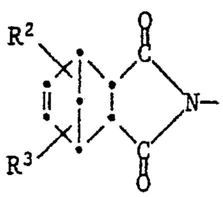

(II),

worin $R^2$ Allyl oder Methallyl bedeutet und $R^3$ Wasserstoff oder eine Methylgruppe ist.

Die Zusammensetzung enthält bevorzugt eine Mischung aus wenigstens einem Imid enthaltend zwei oder drei Reste der Formel II und aus wenigstens einem Acrylatmaterial enthaltend wenigstens eine, bevorzugt wenigstens zwei, besonders bevorzugt zwei bis vier, Gruppen der Formel I.

In dieser Ausführungsform werden insbesondere Imide der Formel III bevorzugt

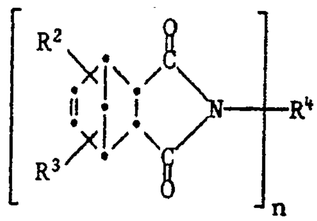 (III),

worin n 1, 2 oder 3 ist,
$R^2$ Allyl oder Methallyl bedeutet,
$R^3$ Wasserstoff oder eine Methylgruppe ist,
$R^4$, im Falle von n = 1, Wasserstoff, eine Hydroxylgruppe oder ein einwertiger organischer Rest mit vorzugsweise 1-40 C-Atomen,
$R^4$, im Falle von n = 2, ein zweiwertiger organischer Rest mit vorzugsweise 2-20 C-Atomen oder
$R^4$, im Falle von n = 3, ein dreiwertiger organischer Rest mit vorzugsweise 6-126 C-Atomen ist.

In Formel III ist $R^2$ bevorzugt Allyl und $R^3$ ist vorzugsweise ein Wasserstoffatom.

Ist n = 1, so handelt es sich bei $R^4$ beispielsweise um einen aliphatischen, cycloaliphatischen, aromatischen oder araliphatischen organischen Rest. Vorzugsweise ist $R^4$ ein Wasserstoffatom, eine Hydroxylgruppe oder ein organischer Rest ausgewählt aus der Gruppe bestehend aus $C_1$-$C_{40}$-Alkyl, das gegebenenfalls durch Sauerstoffatome in der Alkylkette unterbrochen ist, $C_3$-$C_6$Alkenyl, $C_5$-$C_{20}$Cycloalkyl, $C_6$-$C_{20}$Aryl oder Benzyl, wobei jeder dieser Reste gegebenenfalls durch eine Hydroxylgruppe substituiert sein kann, oder einem Rest der Formel IV

$$-Ar^1-X-Ar^2-OH \qquad (IV),$$

worin $Ar^1$ und $Ar^2$ jeweils eine Phenylengruppe sind und X Methylen, Isopropyliden, -CO-, -O-, -S-oder $-SO_2$- ist.

Ist n = 2, so handelt es sich bei $R^4$ beispielsweise um einen aliphatischen, cycloaliphatischen, aromatischen oder araliphatischen Rest. Vorzugsweise ist $R^4$ $C_2$-$C_{20}$Alkylen, das gegebenenfalls durch Sauerstoffatome oder sekundäre Aminogruppen in der Kette substituiert ist, $C_6$-$C_{20}$Arylen oder ein Rest der Formel V

$$-Ar^1-X-Ar^2- \qquad (V),$$

worin $Ar^1$, $Ar^2$ und X die in Formel IV definierte Bedeutung besitzen.

Ist n = 3, so handelt es sich bei $R^4$ beispielsweise um eine aliphatische oder aromatische Gruppe.

Bevorzugt ist $R^4$ dann ein Rest der Formel VI

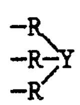 (VI),

worin Y

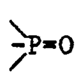 oder 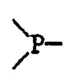

darstellt und R eine $C_2$-$C_{40}$Alkylengruppe ist, die gegebenenfalls in der Kette durch Sauerstoffatome unterbrochen sein kann, eine m- oder p-Phenylengruppe oder eine m- oder p-Phenylenoxygruppe, in der das Sauerstoffatom an die Rest Y gebunden ist, oder
Y ist

$$>CH-$$

und R ist eine m- oder p-Phenylengruppe, oder
Y ist ein Rest

$$CH_3-CH_2-C(CH_2)_3 \text{ oder } -CH_2-CH-CH_2-$$

und R bedeutet eine Oxyalkylengruppe mit zwei oder drei Kohlenstoffatomen, eine Polyoxyethylen- oder Polyoxypropylengruppe mit 4 bis 40 Kohlen-stoffatomen oder eine m- oder p-Phenylenoxygruppe, in der das Sauerstoffatom an die Gruppe Y gebunden ist, oder
Y bedeuten

$$>N-$$

und R ist eine Alkylengruppe mit 2 bis 10 Kohlenstoffatomen, oder eine m- oder p-Phenylengruppe.

Ist $R^4$ eine gegebenenfalls unterbrochene $C_1$-$C_{40}$Alkylgruppe, so handelt es sich um geradkettiges oder verzweigtes Alkyl, beispielsweise um Methyl, Ethyl, Isopropyl, n-Butyl, Isopentyl, n-Hexyl, 2-Ethylhexyl, n-Decyl und n-Dodecyl, besonders bevorzugt jedoch um $C_1$-$C_8$Alkyl.

Ist $R^4$ $C_3$-$C_6$Alkenyl, so kann es ebenfalls geradkettig oder verzweigt sein. Beispiele dafür sind Allyl, Methallyl, 2-Butenyl oder 3-Hexenyl. Allyl wird bevorzugt.

Eine Cycloalkylgruppe $R^4$ ist beispielsweise Cyclopentyl, Cyclohexyl, Cycloheptyl oder Cyclooctyl, wobei Cyclohexyl bevorzugt wird.

Ist $R^4$ Aryl, so handelt es sich dabei beispielsweise um Phenyl, um durch eine oder zwei Methylgruppen substituiertes Phenyl, wie beispielsweise Tolyl oder Xylyl, oder um Naphthyl. Bevorzugt wird Phenyl.

Ist $R^4$ eine hydroxylsubstituierte Alkylgruppe, so handelt es sich dabei beispielsweise um eine $C_1$-$C_{40}$Hydroxyalkylgruppe wie Methylol, 2-Hydroxyethyl, 2-Hydroxypropyl, 3-Hydroxypropyl, 4-Hydroxybutyl, 5-Hydroxypentyl, 2,2-Dimethyl-3-hydroxypropyl, 8-Hydroxyoctyl, 12-Hydroxydodecyl, 16-Hydroxyhexadecyl oder 2,3-Dihydroxypropyl; oder es handelt sich um eine $C_2$-$C_{40}$Hydroxyalkylgruppe, die in der Kette durch Sauerstoffatome unterbrochen ist, wie beispielsweise um 2-Hydroxyethoxyethyl, 3-Hydroxypropoxypropyl und andere Hydroxyalkoxyalkylgruppen der Formel

$$-CH_2-CH_2-(O-CH_2-CH_2\rightarrow_{p},$$

worin p 1 bis 15 ist oder der Formel

$$-CH_2-CH_2-CH_2-(O-CH_2-CH_2-CH_2\rightarrow_{q},$$

worin q 1 bis 10 bedeutet.

Bevorzugte Hydroxyalkylgruppen $R^4$ sind $C_2$-$C_8$Alkylgruppen, die mit einer oder zwei Hydroxylgruppen substituiert sind und gegebenenfalls durch ein bis drei Sauerstoffatome in der Kette unterbrochen sind.

Ist $R^4$ ein hydroxysubstituierter Cycloalkylrest, so kann es sich dabei um eine ein- oder mehrkernige Gruppe handeln, wie beispielsweise um Hydroxycylopentyl, Hydroxycyclohexyl, Hydroxycyclooctyl, Hydroxycyclohexylcyclohexylmethan und 2-Hydroxycyclohexyl-2-cyclohexylpropan, bevorzugt jedoch um eine einkernige Gruppe mit 5 bis 8 Kohlenstoffatomen, insbesondere um Hydroxycyclohexyl.

Bei einer Hydroxyarylgruppe $R^4$ handelt es sich beispielsweise um m-oder p-Hydroxyphenyl oder Hydroxynaphthyl, wobei jeder dieser Reste durch eine oder mehrere $C_1$-$C_4$Alkylgruppen, beispielsweise durch Methyl, Ethyl oder Propyl, substituiert sein kann.

Bevorzugte Hydroxyarylgruppen $R^4$ sind m- oder p-Hydroxyphenyl, wobei das p-Derivat besonders bevorzugt wird.

Bevorzugte Reste $R^4$ der Formel IV sind solche, worin X Methylen, Isopropyliden, -O- oder $-SO_2-$ darstellt.

Ist n = 2, so handelt es sich bei den Alkylengruppen $R^4$ um geradkettige oder verzweigte Reste, wie beispielsweise um Ethylen, 1,2-Propylen, Trimethylen, Tetramethylen, Hexamethylen, Octamethylen oder Dodecamethylen, sowie um Alkylenreste, die in der Kette durch ein oder mehrere Sauerstoffatome oder sekundäre Aminogruppen unterbrochen sind, beispielsweise um Polyoxyethylen-, Polyoxypropylen-und Polyiminoethylengruppen. Bevorzugte Alkylengruppen $R^4$ sind geradkettige $C_2$-$C_{12}$Alkylenreste.
Eine Arylengruppe $R^4$ kann beispielsweise m-Phenylen, p-Phenylen, 1,3-Naphthylen, 1,4-Naphthylen, 1,5-Naphthylen oder 2,6-Naphthylen sein; m- und p-Phenylenreste sind bevorzugt.

Ist $R^4$ eine Gruppe der Formel V, so handelt es sich dabei bevorzugt um einen Rest, worin X Methylen, -O- oder $-SO_2-$ ist, und die freien Valenzen in Formel V sich vorzugsweise in 4- und 4'-Position befinden.

Ist n = 3, so handelt es sich bei den Resten der Formel VI beispielsweise um solche, worin Y

$$>P=O \text{ oder } >P-$$

ist und R Ethylen, 1,2- oder 1,3-Propylen, Tetramethylen, Hexamethylen, Decamethylen, Hexadecamethylen, Ethylenoxy, 1,2- oder 1,3-Propylenoxy, n-Butylenoxy oder n-Hexenylenoxy ist; bevorzugte Reste R sind dabei $C_2$-$C_4$Alkylen, m- oder p-Phenylen oder m- oder p-Phenylenoxy.
Ist Y ist

$$>CH-,$$

so ist R insbesondere eine p-Phenylengruppe. Geeignete Reste der Formel VI, worin Y

$$CH_3-CH_2-C(\overset{|}{C}H_2)_3$$

bedeutet, umfassen Gruppen, worin die Reste R beispielsweise Ethylenoxy, 1,2-oder 1,3-Propylenoxy, eine $C_4$-$C_{20}$Polyoxyethylengruppe oder eine $C_6$-$C_{30}$Polyoxypropylengruppe sind, wobei Reste R der Formel

$$-[CH_2-CH(CH_3)-O]_r$$

mit r = 1, 2 oder 3 besonders bevorzugt werden.

Ist Y im Rest der Formel VI

$$-CH_2-\overset{|}{C}H-CH_2-,$$

so handelt es sich bei den bevorzugten Gruppen R um Ethylenoxy, 1,2- oder 1,3-Propylenoxy, eine $C_4$-$C_{20}$Polyoxyethylengruppe oder eine $C_6$-$C_{30}$Polyoxypropylengruppe, wobei Reste R der Formel

$$-(CH_2-CH_2-O)_r$$

mit r = 1, 2 oder 3 oder m- oder p-Phenylenoxygruppen besonders bevorzugt werden.

Geeignete Gruppen der Formel VI enthalten für den Fall, dass Y

$$>N-$$

ist, als Gruppen R bevorzugt Ethylen, 1,2- oder 1,3-Propylen, 1,4-Butylen oder 1,6-Hexylen, wobei die Reste R insbesondere Ethylen, Trimethylen, m- oder p-Phenylen sind.

Bevorzugte Zusammensetzungen enthalten Imide der Formel III, worin n 1 bedeutet und $R^4$ Wasserstoff, eine Hydroxylgruppe, $C_1$-$C_8$-Alkyl, Allyl, Cyclohexyl, Phenyl, Benzyl, durch ein oder zwei Hydroxylgruppen substituiertes gegebenenfalls in der Kette durch ein bis drei Sauerstoffatome unterbrochenes $C_2$-

$C_8$Alkyl, Hydroxycyclohexyl, Hydroxyphenyl oder eine Gruppe der Formel IV ist, worin X Methylen, Isopropyliden, -O-oder $-SO_2-$ darstellt; oder worin n 2 ist, und $R^4$ geradkettiges $C_2$-$C_{12}$Alkylen, m- oder p-Phenylen oder ein Rest der Formel V bedeutet, worin X Methylen, -O- oder $-SO_2-$ ist; oder worin n 3 ist und $R_4$ eine Gruppe der Formel VI ist, worin Y

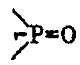 oder 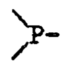

ist und R eine $C_2$-$C_4$Alkylengruppe bedeutet, oder m- oder p-Phenylen oder m- oder p-Phenylenoxy ist, oder worin Y

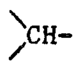

ist und R eine p-Phenylengruppe darstellt oder worin Y

$$CH_3-CH_2-C(CH_2)_3$$

ist, und R eine Gruppe der Formel

$$-[-CH_2-CH(CH_3)-O-]_r-$$

ist und r 1, 2 oder 3 bedeutet, oder worin Y

$$-CH_2-CH-CH_2-$$

ist und R eine Gruppe der Formel

$$-(-CH_2-CH_2-O-)_r-$$

ist und r 1, 2 oder 3 bedeutet oder eine m- oder p-Phenylenoxygruppe ist, oder worin Y

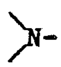

ist und R Ethylen, Trimethylen oder m- oder p-Phenylen darstellt.

Ganz besonders bevorzugte Zusammensetzungen enthalten Imide der Formel III, worin $R^2$ Allyl ist, $R^3$ Wasserstoff bedeutet, und $R^4$, im Falle von n=1, Hydroxyl, Allyl 2-Hydroxyethyl oder p-Hydroxyphenyl bedeutet; oder

$R^4$, im Falle n=2, $-(CH_2)_6-$ oder eine der Gruppen

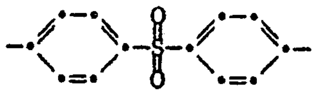 oder 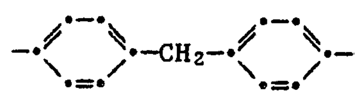

darstellt; oder $R^4$, im Falle von n=3, ein Rest der Formel

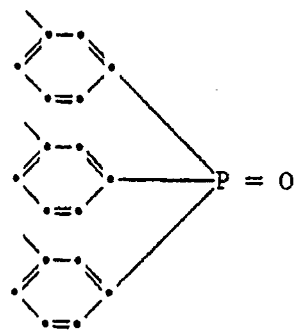

ist.

Es können auch Mischungen von zweien mehreren der oben erwähnten Imide eingesetzt werden.

Ganz besonders bevorzugte Mischungen dieser Erfindung enthalten als Imidkomponente Bis-[4-(allylbicyclo[2.2.1]hept-5-en-2,3-dicarboximidophenyl)]-methan oder eine Mischung dieser Verbindung mit N-Allyl-allylbicyclo[2.2.1]hept-5-en-2,3-dicarboximid und mit N,N'-Hexamethylen-bis(allylbicyclo[2.2.1]hept-5-en-2,3-dicarboximid) oder eine Mischung dieser letztgenannten zwei Verbindungen.

Verbindungen enthaltend den Imidrest der Formel II können nach an sich bekannten Verfahren hergestellt werden, beispielsweise durch Umsetzung eines Anhydrids der Formel VII

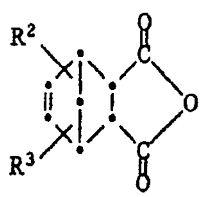
(VII),

worin $R^2$ und $R^3$ die in Formel II definierte Bedeutung besitzen, mit einer Verbindung enthaltend wenigstens eine primäre Aminogruppe; die Umsetzung erfolgt in der Regel bei erhöhter Temperatur.

Die Imide der Formel III können nach an sich bekannten Verfahren hergestellt werden, beispielsweise nach der in der EP-A-105,024 beschriebenen Prozedur. Danach setzt man ein Anhydrid der Formel VII, wie oben definiert, mit einer Verbindung der Formel VIII um

$$R^4 \left( NH_2 \right)_n \qquad \text{(VIII)},$$

worin $R^4$ und n die weiter oben für Formel III definierte Bedeutung besitzen. Die Reaktion erfolgt bei erhöhter Temperatur, wobei das bei der Reaktion gebildete Wasser abdestilliert.

Handelt es sich bei der Verbindung VIII um Ammoniak oder um ein niedrig siedendes Monoamin, so setzt man diese Reaktanden bevorzugt im Ueberschuss ein.

Amine der Formel VIII werden üblicherweise in stöchiometrischen Mengen verwendet.

Die Reaktion kann in An- oder Abwesenheit eines inerten Lösungsmittels durchgeführt werden. Als Lösungsmittel verwendet man insbesondere ein zur azotropen Destillation des Reaktionswassers befähigtes inertes Mittel (Schlepper).

Die Reaktionstemperatur bewegt sich üblicherweise im Bereich von 100 bis 250 °C.

Man kann die Reaktion allerdings auch in der Schmelze unter Anwendung von Druck in der Regel von bis zu 4,500 Pa bei einer Temperatur von 130 bis 220 °C durchführen. Besonders bevorzugte Reaktionstemperaturen bei dieser Variante liegen zwischen 180 und 220 °C.

Imide der Formel III, worin n 3 ist und $R^4$ einen Rest der Formel IV darstellt, worin Y

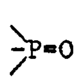 oder 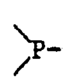

und R eine Alkylenoxy- oder m- oder p-Phenylenoxygruppe ist, können darüber hinaus auch durch Umsetzung eines Imides der Formel III, worin n 1 ist und $R^4$ eine Hydroxyalkyl- oder eine m- oder p-Hydroxyphenylgruppe ist, mit einem Phosphor-(V)-oxidhalogenid oder einem Phosphortrihalogenid, bevorzugt Phosphor-(V)-oxidchlorid, Phosphortribromid oder Phosphortrichlorid, in Gegenwart einer Base, gewöhnlich eines tertiären Amins, hergestellt werden. Die Umsetzung erfolgt in der Regel in einem inerten Lösungsmittel bei Temperaturen von 0 bis 30 °C.

Bevorzugte Komponenten B) sind Acrylatmaterialien mit wenigstens zwei Resten Der Formel I; dies sind Ester einer Carbonsäure enthaltend einen Rest der Formel I mit einem mehrwertigen Alkohol oder Phenol oder einem Epoxidharz.

Die Carbonsäure enthaltend einen Rest der Formel I ist beispielsweise Acrylsäure, Methacrylsäure oder ein Addukt erhältlich durch Umsetzung eines Hydroxyalkylacrylates oder Hydroxyalkylmethacrylates mit einem Polycarbonsäureanhydrid.

Bevorzugte Addukte leiten sich von 2-Hydroxyethyl-, 2-Hydroxypropyl-oder 3-Hydroxypropylacrylat oder -methacrylat an gesättigte oder ungesättigte aliphatische Anhydride, wie beispielsweise Bernsteinsäure-, Adipinsäure-, Glutarsäure- oder Maleinsäureanhydrid, oder an cycloaliphatische Anhydride, wie beispielsweise Tetrahydrophthalsäure-oder Hexahydrophthalsäureanhydrid, oder an aromatische Anhydride, wie beispielsweise Phthalsäure-, Trimellitsäure- oder Pyromellitsäureanhydrid, ab.

Bevorzugte Ester leiten sich von Acrylsäure und Methacrylsäure ab; Beispiele dafür sind Polyacrylsäure- oder -methacrylsäureester von mehrwertigen Alkoholen und Phenolen sowie von Epoxidharzen.

Mehrwertige Alkohole von denen besagte Ester sich ableiten sind beispielsweise solche mit zwei bis zehn alkoholischen Hydroxylgruppen. Solche Alkohole können aliphatische, cycloaliphatische, heterocyclische oder aromatische Verbindungen sein.

Ist der mehrwertige Alkohol aliphatisch, so besitzt er beispielsweise zwei bis sechs Hydroxylgruppen und zwei bis sechzig Kohlenstoffatome. Geeignete Alkohole umfassen Diole, beispielsweise Alkylenglykole, wie Ethylen-, 1,2-Propylen-, Trimethylen-, Tetra-methylen-, Neopentylen-, Hexamethylen-, Octamethylen- oder Dodecamethylenglykole; Oxyalkylenglykole, wie beispielsweise Di-(ethylenglykol) und Di-(propylenglykol); und Addukte der obengenannten Alkylenglykole an ein Mol Ethylenoxid oder Propylenoxid; oder Polyoxyalkylenglykole, beispielsweise Polyoxyethylenglykole, wie Di-, Tri- oder Tetraethylenglykole, und höhere Polyoxyethylenglykole mit bis zu 40 Kohlenstoffatomen; oder Polyoxypropylenglykole, wie beispielsweise Polyoxypropylenglykole mit 6-60 Kohlenstoffatomen, wie Tripropylenglykol; und Addukte der oben erwähnten Alkylenglykole an mehr als ein Mol Ethylenoxid oder Propylenoxid.

Geeignete aliphatische Triole umfassen Glycerin, 1,1,1-Trimethylolpropan und deren Addukte mit Ethylenoxid oder Propylenoxid.

Handelt es sich bei dem aliphatischen Alkohol, von dem sich der Ester ableitet, um ein Tetrol, so kommen beispielsweise Verbindungen wie Erythrit (1,2,3,4-Butantetrol), Pentaerythrit oder deren Addukte mit Ethylenoxid oder Propylenoxid in Frage.

Handelt es sich bei dem aliphatischen Alkohol um ein Pentol, so kann dies beispielsweise Arabit, Xylit oder eines ihrer Addukte mit Ethylenoxid oder Propylenoxid sein.

Zu den geeigneten aliphatischen Hexolen zählen beispielsweise Dipentaerithrit, Mannit, Sorbit und deren Addukte mit Ethylenoxid oder Propylenoxid.

Cycloaliphatische mehrwertige Alkohole, von denen die Ester sich ableiten können, sind in der Regel cycloaliphatische Verbindungen mit zwei bis sechs Hydroxyalkylsubstituenten, beispielsweise einkernige cycloaliphatische Verbindungen mit 5 bis 8 Kohlenstoffatomen, die zwei oder drei Hydroxyalkylsubstituenten besitzen, vorzugsweise ein di- oder tri-hydroxymethyl- oder -hydroxyethylsubstituiertes Cyclohexan.

Leitet sich der mehrwertige Alkohol des Esters von einem heterocyclischen Alkohol ab, so kann die heterocyclische Verbindung beispielsweise zwei bis sechs Hydroxyalkylsubstituenten besitzen; es handelt sich dabei also beispielsweise um einen fünf- oder sechsgliedrigen heterocyclischen Rest mit zwei oder drei Hydroxyalkylsubstituenten, vorzugsweise einen di- oder tri-hydroxymethyl-oder -hydroxyethylsubstituierten fünf- oder sechsgliedrigen Heterocyclus, wie beispielsweise Isocyanursäure, Urazil, Urazol oder Harnsäure; besonders bevorzugt wird Tris-(2-hydroxyethyl)-isocyanurat.

Aromatische mehrwertige Alkohole, von denen die Ester sich ableiten, sind in der Regel aromatische Verbindungen mit zwei bis acht Hydroxyalkylgruppen; dabei kann es sich um einkernige Verbindungen handeln, wie beispielsweise Di- oder Trimethylolphenol, oder um mehrkernige Verbindungen, wie beispielsweise Phenol-Formaldehyd Resole, und Addukte dieser ein- oder mehrkernigen Verbindungen mit Ethylenoxid oder Propylenoxid.

Bevorzugte aromatische Alkohole sind 1,3-Dimethylolbenzol und 1,3,5-Trimethylolbenzol und insbesondere 2,2-Bis-[p-(2-hydroxyethoxy)-phenyl]-propan.

Die Ester dieser mehrwertigen Alkohole sind hauptsächlich bekannt oder können durch an sich bekannte Verfahren hergestellt werden.

Man erhält sie beispielsweise durch Umsetzung der Carbonsäure enthaltend die Gruppe der Formel I, vorzugsweise Acrylsäure oder Methacrylsäure, oder üblicherweise durch Umsetzung mit einem esterbildenden Derivat solcher Carbonsäuren, beispielsweise mit Acrylyl-oder Methacrylylchlorid, mit dem entsprechenden mehrwertigen Alkohol. Enthält letztere Verbindung mehr als zwei Hydroxylgruppen, so können beispielsweise zwei dieser Reste verestert werden und eine oder die übrigen Hydroxylgruppen bleiben unverestert.

Mehrwertige Phenole, von denen die Ester sich ableiten, umfassen einkernige Phenole, wie beispielsweise Resorcin, Bisphenole, wie beispielsweise Bis-(4-hydroxyphenyl)-methan oder 2,2-Bis-(4-hydroxyphenyl)-propan, und Novolake, die sich von Phenol oder einem substituierten Phenol, wie beispielsweise Kresol, und einem Aldehyd, wie beispielsweise Formaldehyd, ableiten.

Die Ester dieser mehrwertigen Phenole sind an sich bekannte Verbindungen oder können nach an sich bekannten Verfahren hergestellt werden. So erhält man sie beispielsweise durch Umsetzung der entsprechenden Phenole mit einem Säurechlorid einer Carbonsäure enthaltend einen Rest der Formel I, vorzugsweise mit Acrylyl- oder Methacrylylchlorid. Die Umsetzung erfolgt in Gegenwart einer Base und wird in der Regel in einem inerten Lösungsmittel unter Erhitzen durchgeführt.

Epoxidharze, von denen die Ester sich ableiten, umfassen Verbindungen mit wenigstens zwei Glycidylgruppen, die an ein Sauerstoffatom, Stickstoffatom oder Schwefelatom gebunden sind, sowie cycloaliphatische Epoxidharze, in denen die Epoxidgruppe Teil des Ringsystems ist.

Zu den Polyglycidylverbindungen zählen beispielsweise Polyglycidylester von aliphatischen, cycloaliphatischen oder aromatischen Polycarbonsäuren, beispielsweise von Adipin-, Bernstein-, Hexahydrophthal- und Phthalsäuren; sowie Poly-N-glycidylverbindungen, beispielsweise Poly-N-glydidylderivate, die sich von aromatischen Aminen, wie Anilin und Bis-(4-aminophenyl)-methan, oder von Hydantoinen, wie 5,5-Dimethylhydantoin, ableiten.

Bevorzugte Epoxidharze sind Polyglycidylether, wie gegebenenfalls vorverlängert sein können, von mehrwertigen Alkoholen oder Phenolen; beispielsweise von den mehrwertigen Alkoholen oder Phenolen, die weiter oben erwähnt sind.

Bevorzugte Polyglycidylether sind Diglycidylether, die gegebenenfalls vorverlängert sein können, von zweiwertigen Alkoholen und Phenolen, beispielsweise diejenigen Diglycidylether, die sich von den weiter oben erwähnten zweiwertigen Alkoholen oder Phenolen ableiten, und Polyglycidylether von Phenol-Aldehyd Novolaken. Besonders bevorzugte Polyglycidylether sind Diglycidylether, die gegebenenfalls vorverlängert sein können, von zweiwertigen Alkoholen mit 2-60 Kohlenstoffatomen oder von Bis-(4-hydroxyphenyl)-methan, 2,2-Bis-(4-hydroxyphenyl)-propan oder 2,2-Bis-(3,5-dibrom-4-hydroxyphenyl)-propan, oder Polyglycidylether von Novolaken auf Basis von Phenol und Formaldehyd.
Die Ester der Epoxidharze können nach an sich bekannten Verfahren hergestellt werden, beispielsweise durch Umsetzung der Carbonsäure enthaltend den Rest der Formel I, bevorzugt Acryl- oder Methacrylsäure, mit dem Epoxidharz in Gegenwart eines tertiären Amins oder eines Oniumsalzkatalysators.

Verbindungen mit nur einer Gruppe der Formel I, also Monoacrylate oder Monomethacrylate, können in der erfindungsgemässen Zusammensetzung in Abwesenheit von oder zusammen mit Verbindungen enthaltend zwei oder mehr Acrylat- oder Methacrylatgruppen verwendet werden. Geeignete monofunktionelle Verbindungen B) umfassen Hydroxyalkylacrylate und -methacrylate, wie beispielsweise 2-Hydroxyethylmethacrylat, oder Allylacrylat und -methacrylat, sowie Addukte von Monoglycidylethern, wie z.B. von Phenylglycidylether oder iso-Octylglycidylether, mit Acryl- oder Methacrylsäure.

Soll die erfindungsgemässe Mischung photopolymerisiert werden, so enthält sie einen Initiator C) der Photopolymerisation für Acrylatmaterialien. Als Initiator der Photopolymerisation C) kann irgendeiner der an sich bekannten Initiatoren der Photopolymerisation von Acrylatmaterialien ausgewählt werden.

Die Komponente C) kann also beispielsweise eine aromatische Carbonylverbindung sein, bevorzugt ein Benzoin, ein Benzoinalkylether, wie beispielsweise der Isopropyl- oder der n-Butylether; ein Benzildialkylketal, beispielsweise Benzildimethylketal' ein $\alpha$-Halogenacetophenon, wie beispielsweise Trichlormethyl-p-tert.-butylphenylketon; ein $\alpha$-Aminoacetophenon, wie beispielsweise Dimethylaminomethylphenylketon und Morpholinomethylphenylketon; ein Dialkoxyacetophenon, wie beispielsweise Diethoxyacetophenon; oder ein Benzophenon, wie beispielsweise Benzophenon oder Bis-(4-dimethylamino)-benzophenon; oder ein Metallocen, bevorzugt ein Titanmetallocen, wie beispielsweise Bis-($\pi$-methylcyclopentadienyl)-bis-($\sigma$-pentafluorphenyl)titan-(IV); oder eine Organometallverbindung eines Elementes der Gruppe IVA, bevorzugt ein Stannan wie beispielsweise Trimethylbenzylstannan, Tributylbenzylstannan oder Dibutylbenzylstannan, in Kombination mit einem photoreduzierbaren Farbstoff, typischerweise Methylenblau oder Bengalrosa; oder ein Chinon, vorzugsweise Anthrachinon oder Camphorchinon in Kombination mit einem Amin, das an einem

aliphatischen α-C-Atom Wasserstoff aufweist, bevorzugt einem tertiären Amin wie z.B. Bis-(4-dimethylamino)-benzophenon und Triethanolamin; oder ein Thioxanthon, bevorzugt ein alkyl- oder halogensubstituiertes Thioxanthon, wie z.B. 2-Isopropylthioxanthon oder 2-Chlorthioxanthon; oder eine aliphatische Dicarbonylverbindung, bevorzugt Diccetyl; oder ein Ketocoumarin, bevorzugt ein Coumarin mit einer carbocyclischen oder heterocyclischen aromatischen Ketogruppe in 3-Position, wie z.B. 3-Benzoyl-7-methoxycoumarin und 3-(4-Cyanbenzoyl)-5,7-dipropoxycoumarin; oder ein Metallcarbonyl, bevorzugt ein Mangancarbonyl so wie beispielsweise Dimangandecacarbonyl; oder ein photoreduzierbarer Farbstoff, typischerweise Methylenblau oder Bengalrosa in Kombination mit einem Reduktionsmittel, bevorzugt einem Elektronendonator wie beispielsweise Benzolsulfinsäure oder anderen Sulfinsäuren oder deren Natriumsalzen, oder einem Arsin, Phosphin oder Thioharnstoff; oder eine Mischung von zwei oder mehreren dieser Komponenten.

Soll die erfindungsgemäss Zusammensetzung direkt oder nach der Photopolymerisation thermisch gehärtet werden, so kann die Mischung noch einen zusätzlichen thermisch aktivierbaren Initiator D) der kationischen Polymerisation enthalten.

Zu den geeigneten Initiatoren dieses Typs zählen beispielsweise Sauerstoffsäuren, bevorzugt phosphorhaltige Säuren, so wie hypophosphorige Säure, Phosphon- und Phosphinsäuren und schwefelhaltige Säuren, so wie aliphatische und insbesondere aromatische Sulfonsäuren und Ester besagter Sauerstoffsäuren, wie beispielsweise Diphenylphosphit; man kann aber auch Lewis-Säuren, wie z.B. Bortrichlorid oder Bortrifluorid und deren Komplexe mit Basen, wie beispielsweise mit Aminen, Amiden oder Phosphinen, einsetzen.

Bevorzugte Initiatoren D) der kationischen Polymerisation sind Verbindungen der Formel IX

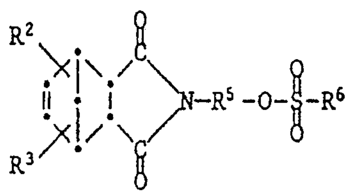 (IX),

worin $R^2$ Allyl oder Methallyl ist,
$R^3$ Wasserstoff oder Methyl bedeutet,
$R^5$ eine direkte Bindung ist oder $C_2$-$C_{20}$Alkylen ist, das gegebenenfalls durch Sauerstoffatome in der Kette unterbrochen sein kann, $C_5$-$C_{20}$Cycloalkylen, $C_6$-$C_{20}$Arylen oder ein Rest der Formel V is und $R^6$ $C_1$-$C_{12}$Alkyl, $C_5$-$C_8$Cycloalkyl, $C_6$-$C_{12}$Aryl oder Benzyl bedeutet.

Bevorzugte Initiatoren der Formel IX sind diejenigen, worin $R^2$ Allyl ist, $R_3$ Wasserstoff bedeutet, $R_5$ eine direkte Bindung ist oder $C_2$-$C_{10}$Alkylen, das gegebenenfalls durch Sauerstoffatome unterbrochen ist, bevorzugt jedoch Ethylen, 2,2-Dimethylpropylen oder einen Rest $-CH_2-CH_2-O-CH_2-CH_2-$ darstellt, oder $C_5$-$C_6$Cycloalkylen, $C_6$-$C_{10}$Arylen oder eine Gruppe der Formel V ist, worin X Methylen oder Isopropyliden darstellt, und $R^6$ $C_1$-$C_6$Alkyl, insbesondere Methyl, oder $C_6$-$C_{10}$Aryl, insbesondere Phenyl, p-Tolyl oder 2-Naphthyl, bedeutet.

Eine ganz besonders bevorzugte Verbindung der Formel IX ist Allylbicyclo[2.2.1]hept-5-en-2,3-dicarbonsäure-N-benzolsulfonyloxyimid.

Initiatoren der Formel IX kann man beispielsweise herstellen, indem man ein Imid der Formel III, worin n 1 ist und $R^4$ $-R^5-OH$ bedeutet, mit einem Sulfonylchlorid der Formel $R^6-SO_2Cl$ unter Kühlung und in Gegenwart eines HCl-Akzeptors, bevorzugt eines tertiären Amins, umsetzt.

Die Reaktion wird üblicherweise in einem inerten Lösungsmittel, wie beispielsweise Toluol, ausgeführt.

Die erfindungsgemässe Zusammensetzung kann auch einen thermisch aktivierbaren Initiator der radikalischen Polymerisation enthalten. Geeignete Initiatoren dieses Typs sind dem Fachmann an sich bekannt und umfassen beispielsweise organische Peroxide, wie z.B. Benzoylperoxid; organische Hydroperoxide, wie z.B. Cumolhydroperoxid, und Azoverbindungen wie z.B. Azo-bis-(isobutyronitril).

Die erfindungsgemässen Zusammensetzungen enthalten das Acrylatmaterial bevorzugt in einer Menge von 10 bis 80 Gew.-%, bezogen auf die Gesamtmenge der Mischung aus Imid(en) und acrylischen Materialien.

Soll die erfindungsgemässe Zusammensetzung bei der Bildherstellung eingesetzt werden, so ist das Acrylatmaterial vorzugsweise in einer Menge von 10 bis 50 Gew.-%, bezogen auf die Gesamtmischung, anwesend.

Der Initiator der Photopolymerisation C) ist in der Regel in einer Menge von 0,1 bis 20 Gew.-%, bevorzugt von 1 bis 10 Gew.-%, bezogen auf die Gesamtmenge an acrylischem Material und Imid(en),

anwesend.

Verwendet man einen Initiator D) der kationischen Polymerisation, so liegt dieser üblicherweise in Mengen von 0,1 bis 15 Gew.-%, bevorzugt von 0,2 bis 5 Gew.-%, bezogen auf das Imidmaterial, vor.

Die erfindungsgemässen Zusammensetzungen können, je nach den physikalischen Eigenschaften der sie aufbauenden Komponenten oder deren Mengenanteilen, flüssig oder fest sein.

Die erfindungsgemässen Zusammensetzungen können übliche Zusätze, wie beispielsweise Füllstoffe, Weichmacher, Pigmente, Farbstoffe, Formtrennmittel oder Flammhemmer enthalten.

Je nach beabsichtigtem Verwendungszweck können die Zusammensetzungen in inerten organischen Lösungsmitteln, wie beispielsweise Toluol, Xylol, Aceton, Methylethylketon, Ethylenglykolmonoalkyl-oder dialkylethern mit ein bis vier Kohlenstoffatomen in den Alkylgruppen, N,N-Dimethylformamid, N,N-Diemthylacetamid, N-Methylpyrrolidon oder Mischungen von zwei oder mehreren dieser Lösungsmittel gelöst sein.

Gegenstand der Erfindung ist auch ein Verfahren zur Herstellung eines polymeren Materials, dadurch gekennzeichnet, dass man eine Zusammensetzung, wie oben definiert, aktinischer Strahlung aussetzt, bis besagte Zusammensetzung photopolymerisiert ist.

Als aktinische Strahlung kann ausschliesslich Ultraviolettstrahlung verwendet werden oder es kann auch eine Kombination von Ultraviolettstrahlung und sichtbarem Licht eingesetzt werden.

Bevorzugt wird aktinische Strahlung im Bereich von 200 bis 600 nm, ganz besonders jedoch im Bereich von 200 bis 400 nm.

Die Auswahl einer geeigneten Strahlungsquelle, die in dem beschriebenen Bereich emittiert, aus den kommerziell erhältlichen Geräten ist für den Fachmann auf dem Gebiet der Polymerisation eine Routinesache.

Geeignete Lichtquellen umfassen Mitteldruck-Quecksilberlampen und Metallhalogenidlampen.

Die jeweils benötigten Belichtunszeiten können vom Fachmann ebenfalls anhand von Routineversuchen ermittelt werden.

Die photopolymerisierten Mischungen können gegebenenfalls weiter durch Wärmebehandlung gehärtet werden. Ein gehärtetes polymeres Material kann also durch Erhitzen einer vorher photopolymerisierten Zusammensetzung erhalten werden.

Die Temperatur und die Härtungszeiten, bei der diese Härtung erfolgt, kann je nach beabsichtigtem Verwendungszweck der ausgehärteten Mischung variieren.

Ueblicherweise wird die photopolymerisierte Mischung auf Temperaturen von 70-300 °C aufgeheizt; in der Regel dauert die Behandlung 10 Minuten bis 6 Stunden.

Soll die erfindungsgemässe Mischung zur Herstellung von Laminaten eingesetzt werden, so wird die photopolymerisierte Zusammensetzung beispielsweise auf Temperaturen zwischen 180 und 300 °C, vorzugsweise zwischen 200 und 170 °C, erhitzt; üblicherweise dauert das Erhitzen zwischen 1 und 4 Stunden.

Soll die erfindungsgemässe Mischung bei der Bildherstellung eingesetzt werden, so kann die photopolymerisierte Mischung auf Wunsch ebenfalls hitzegehärtet werden; üblicherweise erhitzt man hier während 5 Minuten bis zu einer Stunde auf Temperaturen zwischen 80 und 120 °C.

Die Erfindung betrifft ferner ein Verfahren zur Herstellung eines gehärteten polymeren Materials, das gekennzeichnet ist durch das Erhitzen der erfindungsgemässen Mischung bis besagte Mischung ausgehärtet ist.

In einem solchen direkten Hitzehärtungsverfahren wird die Mischung üblicherweise auf 70 bis 300 °C erhitzt, vorzugsweise jedoch auf 180 bis 300 °C und ganz besonders bevorzugt auf 200 bis 270 °C.

Es kann sich als vorteilhaft erweisen, dass man die Härtung anfänglich am unteren Ende dieses Temperaturbereichs durchführt und später die Härtung bei einer oder mehreren höheren Temperaturen innerhalb des angegebenen Bereichs durchführt.

Wünscht man ein gehärtetes Produkt mit einer besonders hohen Festigkeit, so empfiehlt es sich, die Hitzehärtung bis zu 24 Stunden lang durchzuführen. Härtungszeiten können üblicherweise durch Zusatz von Initiatoren D) herabgesetzt werden.

Die erfindungsgemässen Mischungen können polymerisiert und gehärtet werden und führen dann zu Produkten mit hohen Glasübergangstemperaturen und thermischer Stabilität.

Die Mischungen können beispielsweise als Giessharze, Klebstoffe, Laminierharze, Beschichtungen, Enkapsulierharze oder bei der Bildherstellung eingesetzt werden. Wird die erfindungsgemässe Mischung zum Verkleben von Substraten eingesetzt, von denen keines für aktinische Strahlung durchlässig ist, so empfiehlt es sich die erfindungsgemässe Zusammensetzung zunächst aktinischer Strahlung auszusetzen, bis ein photopolymerisierter Film entstanden ist; dieser Film wird dann zwischen die beiden zu verklebenden Substrate gelegt, so dass er in Kontakt mit den zu verklebenden Oberflächen ist, und die Anordnung wird anschliessend durch Hitzebehandlung gehärtet.

Die erfindungsgemässen Zusammensetzungen lassen sich besonders bei der Herstellung von faserverstärkten Harzprepregs und daraus hergestellten ausgehärteten Verbundwerkstoffen einsetzen.

Handelt es sich bei der erfindungsgemässen Mischung um ein festes Material, so kann man das Prepreg durch Imprägnieren besagten fasrigen Verstärkungsmaterials mit einer Lösung besagter Mischung in einem organischen Lösungsmittel herstellen.
Beispiele für Lösungsmittel sind Toluol, Xylol, Methylethylketon, Ethylenglykolmono- und dialkylether und weitere bei der Herstellung von Prepregs übliche Lösungsmittel. Anschliessend wird das Lösungsmittel entfernt. Oder man bringt einen Film der erfindungsgemässen Mischung mit dem fasrigen Verstärkungsmaterial unter Bedingungen zusammen, so dass besagte Mischung das fasrige Material umfliesst und eine zusammenhängende Struktur bildet. Das Prepreg kann dann aktinischer Strahlung ausgesetzt werden, um die Harzmischung zu photopolymerisieren.

Ist die erfindungsgemässe Zusammensetzung in flüssiger Form, so stellt man ein Prepreg vorzugsweise durch Imprägnieren einen faserförmigen Verstärkungsmaterials mit besagter flüssiger Zusammensetzung her und bestrahlt das imprägnierte Material oder erhitzt es bei Temperaturen von 60 bis 160 °C, bis die Zusammensetzung polymerisiert und sich ein im wesentlichen fester, aber noch hitzehärtbarer Film bildet. Eine weitere Methode zur Herstellung von Prepregs aus flüssigen Zusammensetzungen dieser Erfindung umfasst das Bestrahlen oder das Erhitzen auf 60 bis 160 °C der besagten flüssigen Zusammensetzung bis diese zu einem im wesentlichen festen aber noch hitzehärtbaren Film polymerisiert und das anschliessende Kontaktieren von besagten Film mit dem faserigen Verstärkungsmaterial unter solchen Bedingungen, so dass der Film um das fasrige Material herumfliesst und sich eine zusammenhängende Struktur bildet.

Ein gehärteter Verbundwerkstoff kann durch Erhitzen hergestellt werden. Dies erfolgt in der Regel durch Druck und bei Temperaturen von 180 bis 300 °C. Dazu wird ein erfindungsgemässes Prepreg, enthaltend die erfindungsgemässe Zusammensetzung in fester Form, gegebenenfalls zusammen mit anderen Prepregs, oder Schichten aus fasrigen Materialien in der oben beschriebenen Weise gehärtet.

Das faserförmige Verstärkungsmaterial kann in der Form von gewobenem oder nicht gewobenem Tuch als einheitlich ausgerichteter oder zerhackter Faserstrang verwendet werden.

Vorzugsweise handelt es sich bei diesem Fasermaterial um Glas, Kohle, Bor, rostfreiem Stahl, Wolfram, Aluminium, Siliziumcarbid, Asbest, aromatischem Polyamid, Polyethylen oder Polypropylen.

Stellt man ein Prepreg durch Kontaktieren eines Films und des fasrigen Verstärkungsmaterials her, so wird der Film in der Regel durch Anwendung von Hitze und/oder Druck zum Umfliessen des Fasermaterials veranlasst. Dazu kann man beispielsweise erhitzte Platten oder Rollenpaare verwenden. Verwendet man unidirektionale Fasern als Verstärkungsmaterial, so wird vorzugsweise Druck über Rollen in Ausrichtung der Fasern ausgeübt.

Die erfindungsgemässen Zusammensetzungen sind besonders gut in Abbildungsverfahren einsetzbar, beispielsweise bei der Herstellung von Leiterplatten oder gedruckten Schaltungen.
Die Erfindung betrifft daher auch ein Verfahren zur Herstellung einer Abbildung umfassend die Belichtung einer Schicht aus einer erfindungsgemässen Zusammensetzung enthaltend Komponenten A), B) und C) mit aktinischer Strahlung in einem vorbestimmten Muster, bis die Zusammensetzung in den belichteten Flächenanteilen photopolymerisiert ist und Entfernen der Zusammensetzung in den nichtbelichteten Anteilen in an sich bekannter Weise.

Der Ausdruck "Belichtung ... mit aktinischer Strahlung in einem vorbestimmten Muster" umfasst die Belichtung durch ein bildtragende Vorlage und die Belichtung durch einen Energiestrahl, beispielsweise einen Laserstrahl, der z.B. computergesteuert ist und eine Abbildung erzeugt. Die Schicht der photopolymerisierbaren Zusammensetzung kann als Lösung in einem inerten Lösungsmittel auf ein Substrat aufgetragen werden, wobei konventionelle Beschichtungstechniken angewendet werden können.

Beispiele für geeignete Substrate sind Metalle, Kunststoffe oder metallkaschierte Kunststofflaminate.

Nach der bildmässigen Belichtung, die üblicherweise unter Verwendung konventioneller aktinischer Strahlungsquellen, wie oben beschrieben, durchgeführt wird, wird die Entwicklung der Abbildung vorgenommen, wobei die unpolymerisierten Anteile der Zusammensetzung aus den nicht bestrahlten Flächenanteilen der Schicht entfernt werden. Dies geschieht üblicherweise durch Auflösen in einem geeigneten Lösungsmittel, beispielsweise in Ethanol, Aceton oder Gemischen davon oder auch in Gemischen dieser Lösungsmittel mit Wasser.

Die verbliebene photopolymerisierte Zusammensetzung kann auf Wunsch weiter thermisch gehärtet werden. Es resultiert eine Abbildung mit ausgezeichneter thermischer Stabilität.

Die Erfindung wird durch die folgenden Beispiele illustriert, wobei Mengenangaben, soweit nicht anderweitig angegeben, sich auf Gewichtsteile und -prozente beziehen.

Allylbicyclo[2.2.1]hept-5-en-2,3-dicarbonsäure-N-benzolsulfonyloxyimid, das in den Beispielen verwendet wird, stellt man folgendermassen her:

139 g Hydroxylaminhydrochlorid wird in 200 ml Wasser gelöst. Dazu gibt man tropfenweise und unter kräftigem Rühren 408 g Allylbicyclo[2.2.1]hept-5-en-2,3-dicarbonsäuranhydrid und 160 g 50%ige wässrige Natronlauge. Die Mischung wird eine Stunde lang unter Rückfluss gekocht; anschliessend destilliert man das Wasser und Spuren öliger Bestandteile ab, nimmt den Rückstand in Toluol auf, filtriert das Natriumchlorid ab und entfernt das Toluol im Rotationsverdampfer bei 150° C und 2000 pa. Man erhält 402,6 g Allylbicyclo[2.2.1]hept-5-en-2,3-dicarbonsäure-N-hydroxylimid (91,5 % d.Th.) in Form einer hellbraunen viskosen Flüssigkeit mit einer Viskosität von 1,28 Pa•s bei 80° C (Epprecht Viskosimeter).

Analysedaten:

| | berechnet | gefunden |
|---|---|---|
| % C | 65,75 | 65,34 |
| % H | 5,98 | 6,02 |
| % N | 6,39 | 6,40 |

Das N-Hydroxylimid wird in 1 Liter Toluol gelöst, 222,8 g Triethylamin werden zugefügt und es wird gerührt, bis sich eine homogene Lösung gebildet hat.

Diese Lösung wird auf 0° C gekühlt und unter kräftigen Rühren und externem Kühlen werden 324,7 g Benzolsulfonylchlorid tropfenweise so zugegeben, dass die Temperatur der Reaktionsmischung zwischen 5 und 10° C bleibt. Die Mischung wird dann über Nacht bei Raumtemperatur gerührt, Wasser wird zugefügt, der pH-Wert der Mischung wird mit konz. Salzsäure auf 5 eingestellt und die Mischung wird dann zweimal bei 75° C mit Wasser gewaschen.

Nach dem Abtrennen der wässrigen Phase wird das Produkt über $Na_2SO_4$ getrocknet, filtriert und im Rotationsverdmapfer bei 110° C und 2000 Pa konzentriert. Man erhält 471 g einer viskosen Flüssigkeit, die beim Stehen auskristallisiert (F.P.: 97-99° C).

Analysedaten:

| | berechnet | gefunden |
|---|---|---|
| % C | 60,16 | 60,35 |
| % H | 4,77 | 4,85 |
| % N | 3,90 | 3,90 |
| % S | 8,92 | 8,59 |

Im IR-Spektrum misst man Absorptionsbanden bei 575,4; 685,5 und 736,2 $cm^{-1}$ (jeweils Aryl); bei 1195 und 1398 $cm^{-1}$($SO_2$-O-); bei 1620 $cm^{-1}$ (cycl. Doppelbindung); bei 1640 $cm^{-1}$ (Allyldoppelbindung) und bei 1742 $cm^{-1}$ (Carbonylgruppe).

Beispiel 1:

Eine Mischung aus N,N'-Hexamethylenbis(allylbicyclo[2.2.1]hept-5-en-2,3-dicarboximid) (35 Teile) und N-Allyl-allylbicyclo[2.2.1]hept-5-en-2,3-dicarboximid (5 Teile) wird bei 120° C zur Schmelze erhitzt. Man fügt 8 Teile Butandiol-1,4-dimethacrylat zu und lässt die Mischung erkalten. Dazu gibt man 2 Teile Allylmethacrylat, 1 Teil Benzyldimethylketal und 0,2 Teile Allylbicyclo[2.2.1]hept-5-en-2,3-dicarbonsäure-N-benzolsulfonyloxyimid. Die erhaltene Mischung wird als ein 20 Mikrometer dicker Film auf silikonbeschichtetes Papier aufgetragen. Die Schicht wird unter einer 80 W/cm Mitteldruck-Quecksilberlampe im Abstand von 20 cm 30 Sekunden lang bestrahlt und man erhält einen klebfreien Film Dieser Film wird vom silikonbeschichteten Papier abgelöst und 2 Stunden lang bei 250° C erhitzt. Man erhält einen durchgehärteten harten Film mit

einer Glasübergangstemperatur von 285-290 °C (bestimmt durch thermomechanische Analyse; Durchdringung).

Beispiel 2:

Eine Mischung aus 70 Teilen N,N'-Hexamethylenbis-(allylbicyclo[2.2.1]-hept-5-en2,3-dicarboximid) und 10 Teilen N-Allylallylbicyclo[2.2.1]-hept-5-en-2,3-dicarboximid wird bei 120 °C zur Schmelze erhitzt. Man fügt 16 Teile 1,1,1-Trimethylolpropan-trismethacrylat zu und lässt die Mischung abkühlen. Dann mischt man 4 Teile Allylmethacrylat, 2 Teile Benzildimethylketal und 0,4 Teile Allylbicyclo[2.2.1]hept-5-en-2,3-dicarbonsäure-N-benzolsulfonyloxyimid zu. Mit der resultierenden Zusammensetzung wird ein Kohlefasergewebe imprägniert. Das imprägnierte Gewebe besitzt einen Harzgehalt von 65,1 %. Es wird beidseitig mit einer 80 W/cm Mitteldruck-Quecksilberlampe im Abstand von 10 cm 10 Sekunden lang bestrahlt und man erhält ein festes, klebriges Prepreg. Ein Zwölfschichtlaminat wird aus diesen Prepregs hergestellt, indem man 12 10 $cm^2$ grosse Stücke der Prepregs 2 Stunden lang bei 250 °C unter einem Druck von 1,4 $MN/m^2$ verpresst. Das erhaltenen Laminat besitzt einen Harzgehalt von 58,2 % und eine interlaminare Scherfestigkeit von 22,3 $MN/m^2$ bei Raumtemperatur.

Beispiel 3:

Man erhitzt eine Mischung aus 30 Teilen Bis-[4-(allylbicyclo[2.2.1]hept-5-en-2,3-dicarboxyimidophenyl)]-methan, 30 Teilen N,N'-Hexamethylen-bis-(allylbicyclo[2.2.1]hept-5-en-2,3-dicarboxyimid) und 10 Teilen N-Allyl-allylbicyclo[2.2.1]hept-5-en-2,3-dicarboxyimid bei 120 °C zur Schmelze. Dazu gibt man 25 Teile 1,1,1-Trimethylolpropantrismethacrylat und lässt die Mischung abkühlen. Zu der erkalteten Mischung werden 5 Teile Allylmethacrylat, 2 Teile Benzyldimethylketal und 0,4 Teile Allylbicyclo[2.2.1]hept-5-en-2,3-dicarbonsäure-N-benzolsulfonyloxyimid hinzugefügt. Die resultierende Mischung wird zum Imprägnieren eines Kohlefasergewebes verwendet. Das imprägnierte Gewebe besitzt einen Harzgehalt von 63,1 % und wird bestrahlt, wie in Beispiel 2 beschrieben. Man erhält ein klebfreies Prepreg. Daraus stellt man ein 12-schichtiges Laminat her, indem man 12 10 $cm^2$ grosse Stücke der Prepregs übereinanderlegt und 2 Stunden lang bei 250 °C unter einem Druck von 1,4 $MN/m^2$ verpresst. Das resultierende Laminat hat einen Harzgehalt von 58,0 % und eine interlaminare Scherfestigkeit von 21,4 $MN/m^2$ (bei Raumtemperatur) bzw. von 13,5 $MN/m^2$ (bei 135 °C).

Beispiel 4:

Eine Mischung aus 1 Teil Bis-[4-(allylbicyclo[2.2.1]hept-5-en-2,3-dicarboximidophenyl)]-methan, 0,2 Teilen Tris-(2-acryloyloxyethyl)-isocyanurat und 0,05 Teilen 1-Hydroxycyclohexylphenylketon werden in 2 Teilen Dimethylformamid gelöst. Mit dieser Mischung stellt man auf einem kupferkaschierten Laminat eine 24 Mikrometer dicke Schicht her und trocknet fünf Minuten bei 90 °C. Es entsteht ein klebfreier Film. Dieser wird 5 Minuten lang durch ein Negativ mit einer 5000 W Metallhalogenidlampe aus einer Entfernung von 80 cm belichtet. Nach der Entwicklung mit Ethanol erhält man ein negatives Bild.

Beispiel 5:

Man wiederholt Beispiel 4 verwendet aber anstelle des 1-Hydroxcyclohexylphenylketons 0,05 Teile Bis-(4-dimethylamino)-benzophenon. Nach der Entwicklung mit Ethanol erhält man ein negatives Bild.

Beispiel 6:

Man wiederholt Beispiel 4, verwendet aber anstelle von 1-Hydroxycyclohexylphenylketon 0,05 Teile Benzildimethylketal. Nach der Entwicklung mit Ethanol erhält man ein negatives Bild.

Beispiel 7:

Man wiederholt Beispiel 4, verwendet aber anstelle von 1-Hydroxycyclophenylketon 0,05 Teile 2-Isopropylthioxanthon. Nach der Entwicklung mit Ethanol erhält man ein negatives Bild.

Beispiel 8:

Man löst eine Mischung aus 1 Teil Bis-[4-(allylbicyclo[2.2.1]hept-5-en-2,3-dicarboximidophenyl)]-methan, 0,2 Teilen Tris-(2-acryloyloxyethyl)isocyanurat und 0,05 Teilen Bis-($\pi$-methylcyclopentadienyl)-bis-($\sigma$-pentafluorphenyl)-titan-(IV) in einer Mischung aus 1 Teil N-Methylpyrrolidon und 0,45 Teilen Aceton.

Damit stellt man auf einem kupferkaschierten Laminat eine Schicht von 24 Mikrometer Dicke her und trocknet anschliessend 5 Minuten lang bei 90°C, so dass ein klebfreier Film entsteht. Dieser Film wird mittels einer 5000 W Metallhalogenidlampe in einer Entfernung von 80 cm 2 Minuten lang durch ein Negativ belichtet. Der bestrahlte Film wird anschliessend 15 Minuten lang auf 90°C erhitzt und mit einer Mischung aus 90 Vol.% Ethanol und 10 Vol.% Aceton entwickelt. Man erhält ein deutliches negatives Bild.

Die thermogravimetrische Analyse dieses Bildes, d.h., des gehärteten Materials, zeigt, dass unter Stickstoff im Bereich von Raumtemperatur bis 400°C ein Gewichtsverlust von 13,9 Gew.% eintritt (Aufheizgeschwindigkeit: 10°C/Minute) und dass bei einstündigem Erhitzen bei 400°C ein totaler Gewichtsverlust von 31,3 % resultiert.

Beispiel 9:

Eine Mischung aus 1 Teil Bis-[4-(allylbicyclo[2.2.1]hept-5-en-2,3-dicarboximidophenyl)]-methan, 0,2 Teilen Dipentaerythritmonohydroxypentacrylat, 0,05 Teilen Benzildimethylketal und einem Fluorkohlenwasserstoff-Netzmittel (ein Tropfen einer 1%igen Lösung in 2-Butoxyethanol) werden in 1 Teil N-Methylpyrrolidon gelöst.

Daraus stellt man einen 24 Mikrometer dicken Film auf einem Aluminiumblech her, trocknet bei 90°C während 5 Minuten und erhält somit einen klebfreien Film. Dieser wird durch ein Negativ mittels einer 5000 W Metallhalogenidlampe im Abstand von 80 cm 5 Minuten lang bestrahlt. Der bestrahlte Film wird anschliessend 15 Minuten lang auf 90°C erhitzt und dann in einer Mischung aus 90 Vol.% Ethanol und 10 Vol.% Aceton entwickelt. Man erhält ein deutliches negatives Bild.

Beispiel 10:

Eine Mischung aus 1 Teil Bis-[4-(allylbicyclo[2.2.1]hept-5-en-2,3-dicarboximidphenyl)]-methan, 0,2 Teilen Pentaerythrittetraacrylat und 0,05 Teilen 1-Hydroxycyclohexylphenylketon wird in 2 Teilen N-Methylpyrrolidon gelöst. Damit stellt man einen 24 Mikrometer dicken Film auf Weissblech her, trocknet fünf Minuten bei 90°C und erhält somit einen klebfreien Film. Dieser Film wird anschliessend druch ein Negativ mittels einer 5000 W Metallhalogenidlampe in einer Entfernung von 80 cm 5 Minuten lang belichtet. Der belichtete Film wird dan 15 Minuten lang auf 90°C erhitzt und anschliessend in einer Mischung aus 90 Vol.% Ethanol und 10 Vol.% Aceton entwickelt. Man erhält ein deutliches negatives Bild.

Beispiel 11:

Man löst eine Mischung aus 1 Teil Bis-[4-(allylbicyclo[2.2.1]hept-5-en-2,3-dicarboximidophenyl)]-methan, 0,15 Teilen Butandiol-1,4-dimethacrylat und 0,1 Teilen 2-Isopropylthioxanthon in N-Methylpyrrolidon. Mit dieser Lösung stellt man einen 24 Mikrometer dicken Film auf eine kupferkaschierten Laminat her, trocknet 5 Minuten lang bei 90°C und erhält so einen klebfreien Film. Dieser Film wird durch ein Negativ mittels einer 5000 W Metallhalogenidlampe in einer Entfernung von 80 cm 5 Minuten lang bestrahlt. Anschliessend entwickelt man in einer Mischung aus 90 Vol.% Ethanol und 10 Vol.% Wasser und erhält ein deutliches negatives Bild.

Beispiel 12:

Eine Mischung aus 1 Teil Bis-[4-(allylbicyclo[2.2.1]hept-5-en-2,3-dicarboximidophenyl)]-methan, 0,2

Teilen Tris-(2-acryloyloxyethyl)-isocyanurat, 0,01 Teilen Allylbicyclo[2.2.1]hept-5-en-2,3-dicarbonsäure-N-benzolsulfonyloxyimid und 0,05 Teilen Bis-($\pi$-methylcyclopentadienyl)-bis-($\sigma$-pentafluorphenyl)-titan-(IV) wird in einer Mischung aus 2 Teilen N-Methylpyrrolidon und 0,45 Teilen Aceton gelöst.
Aus dieser Lösung stellt man eine 24 Mikrometer dicke Schicht auf einem kupferkaschierten Laminat her, trocknet 5 Minuten lang bei 90 °C und erhält so einen klebfreien Film.
Der Film wird dann durch ein Negativ mittels einer 5000 W Metallhalogenidlampe in einer Entfernung von 80 cm 2 Minuten lang bestrahlt und dann 15 Minuten lang bei 90 °C erhitzt. Man entwickelt mit einem Gemisch aus 90 Vol.% Ethanol und 10 Vol.% Aceton und erhält ein deutliches negatives Bild. Die thermogravimetrische Analyse dieses Bildes unter Stickstoff zeigt folgendes Stabilitätsverhalten des gehärteten Materials:

| Temperaturbereich | Gewichtsverlust (%) |
|---|---|
| Raumtemperatur bis 400°C (10°C/Min.) | 8,7 |
| 1 Stunde bei 400°C | 8,7 |
| Gesamtverlust | 17,4 |

Beispiele 13-24:

Man schmilzt 100 Teile Bis-[4-(allylbicyclo[2.2.1]hept-5-en2,3-dicarboximidophenyl)]-methan bei 150 °C und fügt 0,05 Teile 4-tert.Butyl-1,2-dihydroxybenzol und einen Acrylester zu, so dass ein viskoser Sirup entsteht. Die Viskosität dieser Mischung wird bei 25, 40 oder 80 °C bestimmt.
Man giesst diesen Sirup in Stahlformen von 150x150x4 $mm^3$, deren Oberfläche mit einem Formtrennmittel auf Basis von Silicon behandelt ist. Die Formen werden entgast und die Härtung erfolgt dann nach folgendem Programm:
2 Stunden bei 200 °C, 2 Stunden bei 225 °C und 12 Stunden bei 250 °C. Man erhält harte Platten von ausgezeichneter Qualität. Diese Platten werden zersägt und man erhält Probekörper, an denen physikalische Eigenschaften der gehärteten Mischung ermittelt werden.

Art und Menge der verwendeten Acrylester und gegebenenfalls vorhandene weitere Zusätze sowie die Ergebnisse der Messungen sind in der folgenden Tabelle 1 aufgeführt.

Dabei beziehen sich die Mengenangaben des Acrylesters jeweils auf 100 Teile des Allylnadicimids, $\eta$ bezeichnet die Viskosität in Pa s, $T_v$ is die Temperatur, bei welcher die Viskosität ermittelt wurde, $T_g$ ist die Glasübergangstemperatur, HDT bedeutet die Erweichungstemperatur (gemessen gemäss ISO 75), FS bedeutet die Biegefestigkeit (gemessen gemäss ISO 178), EL bedeutet die Dehnung (gemessen gemäss ISO 178), IS bedeutet die Schlagzähfestigkeit (gemessen gemäss ISO 179) und $WA_r$ bzw. $WA_{100}$ bedeuten die Wasseraufnahme bei Lagerung bei Raumtemperatur (4 Tage) oder bei Lagerung bei 100 °C (1 Stunde), die jeweils in Gewichtsprozenten, bezogen auf den Probekörper, angegeben ist.

Tabelle 1:

| Bsp. | Acrylester und gegebenenfalls weitere Bestandteile | Teile | η Pa s | Tv °C | Tg °C | HDT °C | FS N/mm² | E % | IS KJ/m² | WA r % | WA 100 % |
|---|---|---|---|---|---|---|---|---|---|---|---|
| 13 | TCD-Acrylat 4) | 50 | 5,9 | 25 | 183 | - | 71,5 | 2,1 | 3,5 | - | - |
| 14 | Butandiol-1,4-diacrylat | 50 | 5,9 | 25 | 169 | 185 | 90,0 | 6,9 | 13,0 | 1,9 | 1,4 |
| 15 | Neopentylenglycoldiacrylat | 50 | 1,43 | 40 | - | >250 | 103,4 | 3,9 | 7,46 | 1,0 | 0,65 |
| 16 | Hexandiol-1,6-diacrylat | 50 | 0,38 | 25 | - | 234 | 90,5 | 3,9 | 9,37 | 1,25 | 0,9 |
| 17 1) | Hexandiol-1,6-diacrylat<br>Polymerisationskatalysator 2) | 50<br>1 | - | - | 270 | 252 | 75,7 | 3,6 | 6,29 | 1,45 | 0,6 |
| 18 | Hexandiol-1,6-dimethacrylat | 50 | 1,27 | 25 | 301 | >250 | 74,3 | 3,4 | 6,43 | 0,97 | 0,55 |
| 19 | 2,2'-Di(acryloyloxyethoxy)-bisphenol A | 40 | 0,69 | 80 | - | >250 | 123,3 | 5,2 | 21,08 | 0,40 | 0,43 |
| 20 | 2,2'-Di(acryloyloxyethoxy)-bisphenol A | 50 | 1,35 | 80 | 254 | 149 | 136,2 | 6,0 | 15,1 | 0,46 | 0,38 |
| 21 | 2,2'-Di(methyacryloyloxy-ethoxy)bisphenol A | 50 | 0,77 | 80 | 301 | >250 | 89,1 | 3,5 | 8,64 | 0,84 | 0,52 |
| 22 | Tripropylenglycoldiacrylat | 50 | 0,17 | 80 | 172 | 139 | 104 | 6,8 | 8,17 | 0,98 | 0,81 |
| 23 | Glycerin-tris(propoxyacrylat) | 50 | 0,68 | 80 | - | 234 | 90,6 | 3,9 | 9,37 | 1,25 | 0,89 |
| 24 3) | Glycerin-tris(propoxyacrylat)<br>Polymerisationskatalysator 2) | 45<br>1 | - | - | 293 | 239 | 93,7 | 4,7 | 7,76 | - | - |

1) In Bsp. 17 wird 2 Stunden lang bei 250°C gehärtet.

2) Der Polymerisationskatalysator ist Allylbicyclo[2.21]hept-5-en-2,3-dicarbonsäure-N-benzolsulfonyloxyimid.

3) In Bsp. 24 wird tert.Butylhydroxybenzol durch die gleiche Menge Triethylenglykol-bis-3-(3-tert.butyl-4-hydroxy-5-methylphenyl)-propionat ersetzt und die Härtung erfolgt für 2 Stunden bei 250°C.

4) TCD-Acrylat entspricht der Formel

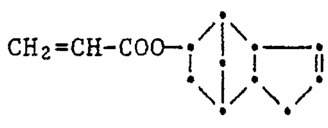 .

## Ansprüche

1. Polymerisierbare Zusammensetzung enthaltend eine Mischung aus
   A) einer Verbindung mit wenigstens einem allyl- oder methallylsubstituierten Bicyclo[2.2.1]hept-5-en-2,3-dicarbonsäureimidrest, und
   B) einer Verbindung, die in einer Menge von 5 bis 95 Gew.-%, bezogen auf die Gesamtmenge der Komponenten A und B vorliegt, mit wenigstens einem polymerisierbaren Rest der Formel I

$$CH_2=C(R^1)-COO- \qquad (I),$$

   worin $R^1$ Wasserstoff oder Methyl ist.

2. Zusammensetzung gemäss Anspruch 1, worin der Imidrest in A) der Formel II entspricht

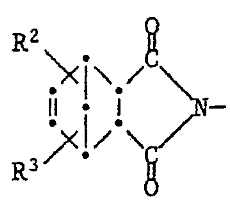 (II),

   worin $R^2$ Allyl oder Methallyl bedeutet und $R^3$ Wasserstoff oder eine Methylgruppe ist.

3. Zusammensetzung gemäss Anspruch 2 enthaltend eine Mischung aus wenigstens einem Imid enthaltend zwei oder drei Reste der Formel II und aus wenigstens einem Acrylatmaterial enthaltend wenigstens eine Gruppe der Formel I.

4. Zusammensetzung gemäss Anspruch 3, worin das Imid eine Verbindung der Formel III ist

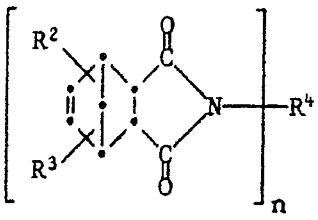

(III),

worin n 1, 2 oder 3 ist,
$R^2$ Allyl oder Methallyl bedeutet,
$R^3$ Wasserstoff oder eine Methylgruppe ist,
$R^4$, im Falle von n = 1, Wasserstoff, eine Hydroxylgruppe oder ein einwertiger organischer Rest,
$R^4$, im Falle von n = 2, ein zweiwertiger organischer Rest oder
$R^4$, im Falle von n = 3, ein dreiwertiger organischer Rest ist.

**5.** Zusammensetzung gemäss Anspruch 4, worin $R^2$ Allyl bedeutet und $R^3$ Wasserstoff ist.

**6.** Zusammensetzung gemäss den Ansprüchen 4 oder 5, worin n = 1 ist und $R^4$ ein Wasserstoffatom, eine Hydroxylgruppe oder ein organischer Rest ist, der ausgewählt wird aus der Gruppe bestehend aus $C_1$-$C_{40}$-Alkyl, das gegebenenfalls durch Sauerstoffatome in der Alkylkette unterbrochen ist, $C_3$-$C_6$Alkenyl, $C_5$-$C_{20}$Cycloalkyl, $C_6$-$C_{20}$Aryl oder Benzyl, wobei jeder dieser Rests gegebenenfalls durch eine Hydroxylgruppe substituiert sein kann, oder einem Rest der Formel IV

$$-Ar^1-X-Ar^2-OH \qquad (IV),$$

worin $Ar^1$ und $Ar^2$ jeweils eine Phenylengruppe sind und X Methylen, Isopropyliden, -CO-, -O-, -S- oder $-SO_2-$ ist; oder worin n = 2 ist und $R^4$ $C_2$-$C_{20}$Alkylen, das gegebenenfalls durch Sauerstoffatome oder sekundäre Aminogruppen in der Kette substituiert ist, $C_6$-$C_{20}$Arylen oder ein Rest der Formel V ist

$$-Ar^1-X-Ar^2- \qquad (V),$$

worin $Ar^1$, $Ar^2$ und X die in Formel IV definierte Bedeutung besitzen; oder worin n = 3 ist und $R^4$ ein Rest der Formel VI ist

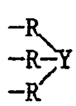

(VI),

worin Y

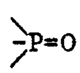 oder 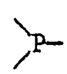

darstellt und R eine $C_2$-$C_{40}$Alkylengruppe ist, die gegebenenfalls in der Kette durch Sauerstoffatome unterbrochen sein kann, eine m- oder p-Phenylengruppe oder eine m- oder p-Phenylenoxygruppe, in der das Sauerstoffatom an den Rest Y gebunden ist, oder
Y

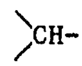

ist und R eine m- oder p-Phenylengruppe ist, oder
Y ein Rest

$$CH_3-CH_2-C-(CH_2)_3 \text{ oder } -CH_2-CH-CH_2-$$

ist und R eine Oxyalkylengruppe mit zwei oder drei Kohlenstoffatomen, eine Polyoxyethylen- oder Polyoxypropylengruppe mit 4 bis 40 Kohlenstoffatomen oder ein m- oder p-Phenylenoxygruppe, in der das Sauerstoffatom an die Gruppe Y gebunden ist bedeutet, oder
Y

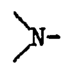

bedeutet und R eine Alkylengruppe mit 2 bis 10 Kohlenstoffatomen, oder eine m- oder p-Phenylengruppe ist.

**7.** Zusammensetzung gemäss den Ansprüchen 3 bis 6, worin das Acrylatmaterial ein Ester einer Carbonsäure, die eine Gruppe der Formel I enthält, mit einem mehrwertigen Alkohol oder Phenol oder mit einem Epoxidharz ist.

**8.** Zusammensetzung gemäss den Ansprüchen 1 bis 7 enthaltend als zusätzliche Komponente C) einen Initiator der Photopolymerisation für Acrylatmaterialien.

**9.** Zusammensetzung gemäss den Ansprüchen 1 bis 8, enthaltend als zusätzliche Komponente D) einen thermisch aktivierbaren Initiator der kationischen Polymerisation.

**10.** Zusammensetzung gemäss Anspruch 9, worin D) eine Verbindung der Formel IX ist

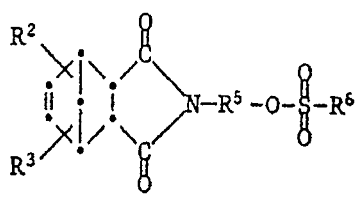

(IX),

worin $R^2$ Allyl oder Methallyl ist,
$R^3$ Wasserstoff oder Methyl bedeutet,
$R^5$ eine direkte Bindung ist oder $C_2$-$C_{20}$Alkylen ist, das gegebenenfalls durch Sauerstoffatome in der Kette unterbrochen sein kann, $C_5$-$C_{20}$Cycloalkylen, $C_6$-$C_{20}$Arylen oder ein Rest der Formel V gemäss Anspruch 6 ist, und
$R^6$ $C_1$-$C_{12}$Alkyl, $C_5$-$C_8$Cycloalkyl, $C_6$-$C_{12}$Aryl oder Benzyl bedeutet.

**11.** Zusammensetzung gemäss den Ansprüchen 1 bis 10,, worin Komponente B) 10 bis 80 Gew.%, bezogen auf die Menge von A) und B), ausmacht.

**12.** Verfahren zur Herstellung eines polymeren Materials, dadurch gekennzeichnet, dass man eine Zusammensetzung gemäss den Ansprüchen 1 bis 11 aktinischer Strahlung aussetzt, bis besagte Zusammensetzung photopolymerisiert ist.

**13.** Verfahren zur Herstellung eines gehärteten polymeren Materials, dadurch gekennzeichnet, dass man eine Zusammensetzung gemäss den Ansprüchen 1 bis 11 erhitzt, bis besagte Zusammensetzung

ausgehärtet ist.

**14.** Verwendung einer Zusammensetzung gemäss den Ansprüchen 1 bis 11 zur Herstellung von Prepregs.

**15.** Verfahren zur Herstellung von Abbildungen umfassend die Belichtung einer Schicht aus einer Zusammensetzung gemäss den Ansprüchen 8 bis 11 mit aktinischer Strahlung in einem vorbestimmten Muster, bis die Zusammensetzung in den belichteten Flächenanteilen photopolymerisiert ist und Entfernen der Zusammensetzung in den nichtbelichteten Anteilen in an sich bekannter Weise.

## Claims

**1.** A polymerizable composition comprising a mixture of
A) a compound having at least one allyl- or methallyl-substituted bicyclo[2.2.1]hept-5-ene-2,3-dicarboximide radical, and
B) a compound, which is present in an amount of from 5 to 95 % by weight, based on the total amount of components A and B, having at least one polymerizable radical of the formula I

$$CH_2=C(R^1)-COO- \qquad (I),$$

in which $R^1$ is hydrogen or methyl.

**2.** A composition according to claim 1, in which the imide radical in A) has the formula II

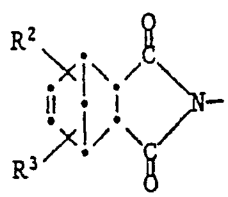

(II),

in which $R^2$ is allyl or methallyl and $R^3$ is hydrogen or a methyl group.

**3.** A composition according to claim 2 comprising a mixture of at least one imide comprising two or three radicals of the formula II and at least one acrylate material comprising at least one group of the formula I.

**4.** A composition according to claim 3, in which the imide is a compound of the formula III

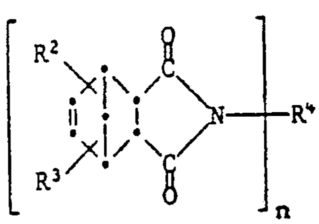

(III),

in which n is 1, 2 or 3,
$R^2$ is allyl or methallyl,
$R^3$ is hydrogen or a methyl group,
when n is 1, $R^4$ is hydrogen, a hydroxyl group or a monovalent organic radical,
when n is 2, $R^4$ is a divalent organic radical or
when n is 3, $R^4$ is a trivalent organic radical.

5. A composition according to claim 4, in which $R^2$ is allyl and $R^3$ is hydrogen.

6. A composition according to either claim 4 or claim 5, in which n is 1 and $R^4$ is a hydrogen atom, a hydroxyl group or an organic radical selected from the group consisting of $C_1$-$C_{40}$alkyl which is optionally interrupted by oxygen atoms in the alkyl chain, $C_3$-$C_6$alkenyl, $C_5$-$C_{20}$cycloalkyl, $C_6$-$C_{20}$aryl or benzyl, where any of these radicals may be unsubstituted or substituted by a hydroxyl group or a radical of the formula IV

$$-Ar^1-X-Ar^2-OH \qquad (IV),$$

in which $Ar^1$ and $Ar^2$ are each a phenylene group and X is methylene, isopropylidene, -CO-, -O-, -S- or $-SO_2-$; or in which n is 2 and $R^4$ is $C_2$-$C_{20}$alkylene which is optionally substituted in the chain by oxygen atoms or secondary amino groups, $C_6$-$C_{20}$arylene or a radical of the formula V

$$-Ar^1-X-Ar^2- \qquad (V),$$

in which $Ar^1$, $Ar^2$ and X are as defined in the formula IV; or in which n is 3 and $R^4$ is a radical of the formula VI

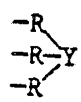 (VI),

in which Y is

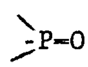 or 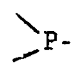

and R is a $C_2$-$C_{40}$alkylene group, which may be optionally interrupted in the chain by oxygen atoms, a m- or p-phenylene group or a m- or p-phenyleneoxy group in which the oxygen atom is bonded to the radical Y, or
Y is

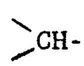

and R is a m- or p-phenylene group, or
Y is a

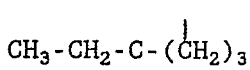 or 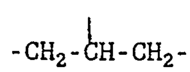

radical and R is an oxyalkylene group of two or three carbon atoms, a polyoxyethylene or polyoxypropylene group of 4 to 40 carbon atoms or a m- or p-phenyleneoxy group in which the oxygen atom is bonded to the group Y, or Y is

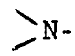

and R is an alkylene group of 2 to 10 carbon atoms or a m- or p-phenylene group.

**7.** A composition according to any one of claims 3 to 6, in which the acrylate material is an ester of a carboxylic acid comprising a group of the formula I with a polyhydric alcohol or phenol or with an epoxy resin.

**8.** A composition according to any one of claims 1 to 7 comprising as additional component C) a photopolymerization initiator for acrylate materials.

**9.** A composition according to any one of claims 1 to 8, comprising as additional component D) a thermally activated cationic polymerization initiator.

**10.** A composition according to claim 9, in which D) is a compound of the formula IX

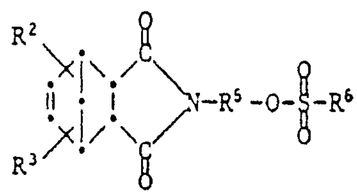

(IX),

in which $R^2$ is allyl or methallyl,
$R^3$ is hydrogen or methyl,
$R^5$ is a direct bond or is $C_2$-$C_{20}$alkylene which may be optionally interrupted by oxygen atoms in the chain, $C_5$-$C_{20}$cycloalkylene, $C_6$-$C_{20}$-arylene or a radical of the formula V according to claim 6, and $R^6$ is $C_1$-$C_{12}$alkyl, $C_5$-$C_8$cycloalkyl, $C_6$-$C_{12}$aryl or benzyl.

**11.** A composition according to any one of claims 1 to 10, in which component B) is present in an amount of from 10 to 80% by weight, based on the amount of A) and B).

**12.** A process for the preparation of a polymeric material which comprises exposing a composition according to any one of claims 1 to 11 to actinic radiation until said composition is photopolymerized.

**13.** A process for the preparation of a cured polymeric material which comprises heating a composition according to any one of claims 1 to 11 until said composition is cured.

**14.** Use of a composition according to any one of claims 1 to 11 for the production of prepregs.

**15.** A process for the production of an image comprising exposing a layer of a composition according to any one of claims 8 to 11 to actinic radiation in a predetermined pattern until the composition is photopolymerized in the exposed areas and removing the composition in the unexposed areas in a known manner.

## Revendications

**1.** Composition photopolymérisable qui renferme un mélange constitué :

A) d'un composé contenant au moins un radical d'allyl- ou de méthallyl-bicyclo[2.2.1]heptène-5 dicarboximide-2,3 et

B) d'un composé présent en une quantité de 5 à 95% en poids, par rapport à la somme des poids des composantes A et B, qui contient au moins un radical polymérisable répondant à la formule I :

$$CH_2=C(R^1)-COO- \qquad (I)$$

dans laquelle $R^1$ représente l'hydrogène ou un radical méthyle.

2. Composition selon la revendication 1 dans laquelle le radical imide contenu dans A) répond à la formule II :

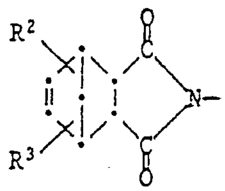

(II),

dans laquelle $R^2$ représente un radical allyle ou méthallyle et $R^3$ représente l'hydrogène ou un radical méthyle.

3. Composition selon la revendication 2 qui renferme un mélange constitué d'au moins un imide contenant deux ou trois radicaux de formule II et d'au moins une matière acrylique contenant au moins un radical de formule I.

4. Composition selon la revendication 3 dans laquelle l'imide est un composé répondant à la formule III:

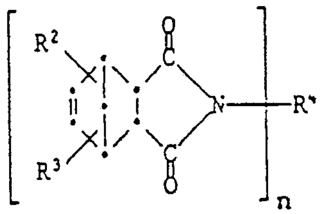

(III),

dans laquelle
n est égal à 1, à 2 ou à 3,
$R^2$ représente un radical allyle ou méthallyle,
$R^3$ représente l'hydrogène ou un radical méthyle, et
$R^4$ représente :
- dans le cas où n est égal à 1, l'hydrogène, un radical hydroxy ou un radical organique univalent,
- dans le cas où n est égal à 2, un radical organique bivalent,
- dans le cas où n est égal à 3, un radical organique trivalent.

5. Composition selon la revendication 4 dans laquelle $R^2$ représente un radical allyle et $R^3$ l'hydrogène.

6. Composition selon l'une des revendications 4 et 5 dans laquelle :
- ou bien n est égal à 1
et $R^4$ représente un atome d'hydrogène, un radical hydroxy ou un radical organique pris dans l'ensemble constitué par les alkyles en $C_1$-$C_{40}$ dont la chaîne alkyle est éventuellement interrompue par des atomes d'oxygène, les alcényles en $C_3$-$C_6$, les cycloalkyles en $C_5$-$C_{20}$, les aryles en $C_6$-$C_{20}$ et le benzyle, chacun de ces radicaux étant éventuellement porteur d'un hydroxy, ainsi que les radicaux répondant à la formule IV

$$-Ar^1-X-Ar^2-OH \qquad (IV)$$

dans laquelle $Ar^1$ et $Ar^2$ représentent chacun un radical phénylène et X représente un radical

méthylène, isopropylidène, -CO-, -O-, -S- ou $-SO_2-$,

- ou bien n est égal à 2
  et $R^4$ représente un alkylène en $C_2$-$C_{20}$ dont la chaîne est éventuellement interrompue par des atomes d'oxygène ou des radicaux amino secondaires, un arylène en $C_6$-$C_{20}$ ou un radical répondant à la formule V :

$$-Ar^1-X-Ar^2- \qquad (V)$$

  dans laquelle $Ar^1$, $Ar^2$ et X ont les significations qui leur ont été données à propos de la formule IV,
- ou bien n est égal à 3
  et $R^4$ représente un radical répondant à la formule VI :

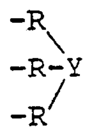

(VI)

dans laquelle
Y représente un radical

$$>P=O \text{ ou } >P-$$

et R représente un alkylène en $C_2$-$C_{40}$ dont la chaîne est éventuellement interrompue par des atomes d'oxygène, un radical m- ou p-phénylène, ou un radical m- ou p-phénylèneoxy dont l'atome d'oxygène est lié au radical Y, ou
Y représente un radical

$$>CH-$$

et R un radical m- ou p-phénylène, ou
Y représente un radical

$$CH_3-CH_2-C(CH_2)_3$$

ou

$$-CH_2-CH-CH_2-$$

et R représente un radical oxy-alkylène à deux ou trois atomes de carbone, un radical poly-oxy-éthylène ou poly-oxy-propylène contenant de 4 à 40 atomes de carbone ou un radical m- ou p-phénylène-oxy dont l'atome d'oxygène est lié au radical Y, ou
Y représente un radical

$$>N-$$

et R représente un radical alkylène contenant de 2 à 10 atomes de carbone ou un radical m- ou p-phénylène.

**7.** Composition selon l'une quelconque des revendications 3 à 6, dans laquelle la matière acrylique est un ester dérivant, d'une part, d'un acide carboxylique contenant un radical de formule I et, d'autre part, d'un poly-alcool ou d'un poly-phénol ou d'une résine époxydique.

**8.** Composition selon l'une quelconque des revendicatìcns 1 à 7, qui contient comme composantes supplémentaires C) un amorceur de photopolymérisation pour les matières acryliques.

**9.** Composition selon l'une quelconque des revendications 1 à 8, qui contient comme composantes supplémentaires D) un amorceur thermo-activable pour la polymérisation cationique.

**10.** Composition selon la revendication 9 dans laquelle D) est un composé répondant à la formule IX :

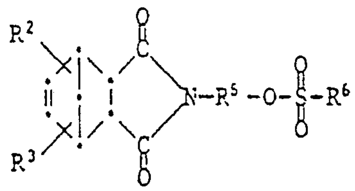
(IX),

dans laquelle
- $R^2$ représente un allyle ou un méthallyle,
- $R^3$ représente l'hydrogène ou un méthyle,
- $R^5$ représente une liaison directe, un alkylène en $C_2$-$C_{20}$ dont la chaîne est éventuellement interrompue par des atomes d'oxygène, un cycloalkylène en $C_5$-$C_{20}$, un arylène en $C_6$-$C_{20}$ ou un radical de formule V selon la revendication 6, et
- $R^6$ représente un alkyle en $C_1$-$C_{12}$, un cycloalkyle en $C_5$-$C_8$, un aryle en $C_6$-$C_{12}$ ou un benzyle.

**11.** Composition selon l'une quelconque des revendications 1 à 10, dans laquelle la composante B) représente de 10 à 80% en poids par rapport à la quantité de A) et B).

**12.** Procédé pour préparer une matière polymère, procédé caractérisé en ce qu'on expose à un rayonnement actinique une composition selon l'une quelconque des revendications 1 à 11 jusqu'à ce que ladite composition soit photopolymérisée.

**13.** Procédé pour préparer une matière polymère durcie, procédé caractérisé en ce qu'on chauffe une composition selon l'une quelconque des revendications 1 à 11 jusqu'à ce que ladite composition soit durcie.

**14.** Application d'une composition selon l'une quelconque des revendications 1 à 11 à la fabrication de pré-imprégnés.

**15.** Procédé pour réaliser des images, procédé selon lequel on irradie au moyen d'un rayonnement actinique, conformément à un modèle donné, une couche d'une composition selon l'une quelconque des revendications 8 à 11, cela jusqu'à ce que la composition soit photopolymérisée aux endroits de la surface qui ont été touchés par le rayonnement et on élimine la composition aux endroits qui n'ont pas été touchés par le rayonnement, de manière connue.